# FASCICULE DE BREVET EUROPEEN

(11) **EP 2 938 595 B1**
(45) Date de publication et mention de la délivrance du brevet: **14.06.2017**
(21) Numéro de dépôt: 13814987.7
(22) Date de dépôt: 27.12.2013
(51) Int. Cl.: C07D 211/06

(54) **PROCEDE DE SYNTHESE D'UNE HYDRAZINE UTILE DANS LE TRAITEMENT DU VIRUS DU PAPILLOME**
VERFAHREN ZUR SYNTHESE EINES HYDRAZIN ZUR BEHANDLUNG DES PAPILLOMAVIRUS
METHOD FOR THE SYNTHESIS OF A HYDRAZINE THAT CAN BE USED IN THE TREATMENT OF THE PAPILLOMA VIRUS

(30) Priorité: 27.12.2012 FR 1262872
(43) Date de publication de la demande: 04.11.2015
(73) Titulaire: Anaconda Pharma, 94800 Villejuif (FR)
(72) Inventeur: BLUMENFELD, Marta, F-75013 Paris (FR); COMPERE, Delphine, F-92330 Sceaux (FR); CIUFOLINI, Marco A., Vancouver, BC V6K 1V5 (CA)
(74) Mandataire: Regimbeau
(86) Numéro de dépôt international: PCT/EP2013/078035
(87) Numéro de publication internationale: WO 2014/102313

(56) Documents cités:
- US-A1- 2009 209 586
- JOËLLE VIRET ET AL: "Practical synthesis of optically active á-hydrazino acids from á-amino acids", TETRAHEDRON, ELSEVIER SCIENCE PUBLISHERS, AMSTERDAM, NL, vol. 43, no. 5, 1987, pages 891-894, XP008136595, ISSN: 0040-4020, DOI: 10.1016/S0040-4020(01)90026-2 cité dans la demande

## Description

La présente invention concerne un nouveau procédé de synthèse de composés utiles pour le traitement du virus du papillome décrits notamment dans la demande WO 2007/135 106.

Les composés décrits dans la demande WO 2007/135 106 ont la structure suivante : 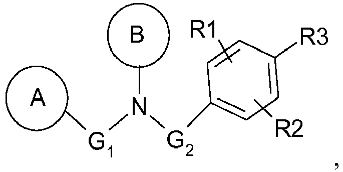 pour laquelle :
- A représente un groupe aryle, cycloalkyle, cycloalkényle ou hétérocycle éventuellement substitué,
- B représente un aryle ou un hétérocycle éventuellement substitué,
- R1 et R2 représentent indépendamment un atome d'hydrogène ou divers substituants,
- R3 représente une fonction acide ou un radical prodrogue ou un bioisostère de cette fonction,
- G₁ représente une liaison ou une chaîne hydrocarbonée, et
- G₂ représente un groupe 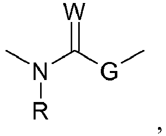 avec R représentant un atome d'hydrogène ou divers substituants, W représentant O, S ou NH et G représentant une liaison ou une chaîne hydrocarbonée.

Le procédé de synthèse de ces composés décrit dans la demande WO 2007/135 106 passe par une hydrazine, comme intermédiaire de synthèse, de structure suivante : 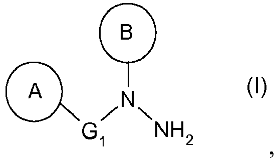 qui est elle-même préparée à partir de l'amine de structure suivante :

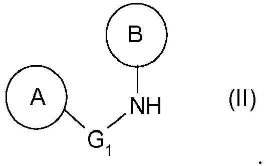

La préparation de l'hydrazine à partir de l'amine correspondante est réalisée par nitrosation de l'amine en présence de NaNO₂ pour donner le composé N-nitroso correspondant qui est ensuite réduit pour donner l'hydrazine. Cependant, les composés nitroso étant dangereux d'utilisation, une telle voie de synthèse n'est pas la plus adaptée d'un point de vue industriel. De plus, des normes assez strictes concernant les teneurs résiduelles acceptables de composé nitroso dans un produit pharmaceutique conduisent à envisager une autre voie de synthèse.

Les inventeurs ont ainsi découvert qu'il était possible de préparer l'hydrazine désirée à partir de l'amine correspondante en passant par une urée intermédiaire et non par un composé N-nitroso.

La présente invention a donc pour objet un procédé de préparation d'une hydrazine de formule générale (I) : 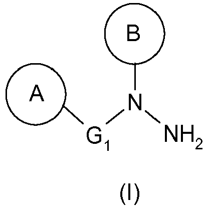 et de ses sels pharmaceutiquement acceptables, dans lequel :
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle, de préférence identiques,
▪ A représente un groupement aryle, tel que phényle, éventuellement substitué :
   - en position méta ou para par :
      - un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
      - un groupement cycloalkyle, aryle ou arylalkyle, chacun éventuellement substitué par un ou deux substituants, identiques ou différents, tels qu'un groupement acyle ou alkoxy,
   - et/ou en position ortho ou méta par un groupement alkyle, et
▪ B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle,
à partir d'une amine de formule (II) : 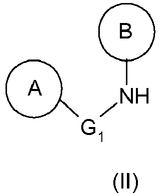 dans laquelle G₁, A et B sont tels que définis ci-dessus,
caractérisé en ce que ledit procédé passe par une urée de formule (III) comme intermédiaire de synthèse : 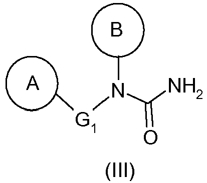 dans laquelle G₁, A et B sont tels que définis ci-dessus.

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; et
(3) les sels formés lorsqu'un proton acide présent dans le composé parent soit est remplacé par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Par « insaturé », on entend, au sens de la présente invention, que le groupement comprend une ou plusieurs insaturations.

Par « insaturation », on entend, au sens de la présente invention une double liaison ou une triple liaison.

Par « halogène », on entend, au sens de la présente invention, un atome de fluor, de brome, de chlore ou d'iode. Avantageusement, il s'agit d'un atome de fluor, de brome ou de chlore.

Par groupement « alkyle », on entend, au sens de la présente invention, une chaîne hydrocarbonée saturée, linéaire ou ramifiée, comportant de 1 à 6 atomes de carbone, en particulier les groupes méthyle, éthyle, n-propyle, isopropyle, n-butyle, iso-butyle, sec-butyle, tert-butyle, n-pentyle ou encore n-hexyle. Avantageusement, il s'agit du méthyle.

Par groupement « cycloalkyle », on entend, au sens de la présente invention, un système monocyclique ou polycyclique, de préférence mono-, bi- ou tri-cyclique, saturé, comportant de 3 à 12 atomes de carbone, les cycles pouvant être deux à deux fusionnés ou pontés, tel que les groupes cyclopropyle, cyclopentyle, cyclohexyle, cycloheptyle, cyclooctyle, adamantyle, décalinyle ou encore norbornyle.

Par groupement « N-cycloalkyle », on entend, au sens de la présente invention, un groupe cycloalkyle tel que défini ci-dessus pour lequel un atome de carbone a été substitué par un atome d'azote, la liaison avec la molécule se faisant par cet atome d'azote. Il s'agit avantageusement d'un groupe pipéridin-1-yle ou pyrrolidin-1-yle.

Par groupement « acyle », on entend, au sens de la présente invention, un groupe de formule -C(O)-Z, où Z représente un groupe alkyle tel que défini ci-dessus ou un phényle. Il peut s'agir avantageusement d'un groupe acétyle, éthylcarbonyle ou encore benzoyle.

Par groupement « alkoxy », on entend, au sens de la présente invention, un groupe alkyle tel que défini ci-dessus lié à la molécule par l'intermédiaire d'un atome d'oxygène. Il peut s'agir en particulier d'un groupe méthoxy, éthoxy, n-propoxy, isopropoxy, n-butoxy, iso-butoxy, sec-butoxy ou encore tert-butoxy.

Par groupement « halogénoalkoxy », on entend, au sens de la présente invention, un groupe alkoxy tel que défini ci-dessus substitué par un ou plusieurs atome(s) d'halogène tel(s) que défini(s) ci-dessus. De préférence, il s'agira de fluoroalkoxy, c'est-à-dire d'un groupe alkoxy substitué par un ou plusieurs atome(s) de fluor, tel qu'un groupe -OCF₃ ou encore -OCH₂CF₃.

Par groupement « aryle », on entend, au sens de la présente invention, un groupement aromatique, comportant de préférence de 5 à 10 atomes de carbone et comprenant un ou plusieurs cycles accolés, comme par exemple un groupement phényle ou naphtyle. Avantageusement, il s'agit du phényle.

Par « hétérocycle », on entend, au sens de la présente invention, un système monocyclique ou polycyclique, et de préférence mono- ou bi-cyclique, saturé, insaturé ou aromatique, comportant de 3 à 12 chaînons, les cycles pouvant être deux à deux fusionnés, spiro-fusionnés ou pontés, et comprenant 1 à 4 hétéroatomes, identiques ou différents, choisis parmi O, S et N, et comprenant éventuellement un ou deux groupements oxo ou thioxo, étant compris que dans le cas d'un système polycyclique, l'un des cycles peut être aromatique tandis que le ou les autres peuvent être aromatique, saturé ou insaturé. Avantageusement, il s'agira des groupes pipéridyle, pipérazyle, furyle, thiényle, pyrrolyle, pyrazolyle, imidazolyle, pyridyle, pyrimidyle, pyrazinyle, pyradizinyle, benzofuryle, benzothiényle, indolyle, quinolyle, isoquinolyle, benzodioxolyle, benzodioxinyle, benzo[1,2,5]thiadiazolyle, benzo[1,2,5]oxadiazolyle, [1,2,3]triazolyle et [1,2,4]triazolyle.

Par groupement « arylalkyle », on entend, au sens de la présente invention, un groupe aryle tel que défini ci-dessus lié à la molécule par l'intermédiaire d'un groupe alkyle tel que défini ci-dessus. De préférence, il s'agit d'un groupe benzyle.

Par « acylaminoalkyle », on entend, au sens de la présente invention, un groupement de formule -Alk-NHCO-Alk', où Alk et Alk' représentent, indépendamment l'un de l'autre, un groupe alkyle tel que défini ci-dessus.

L'hydrazine de formule (I) telle que définie ci-dessus pourra ainsi être préparée à partir de l'urée de formule (III) telle que définie ci-dessus par un réarrangement de Shestakov.

Cette réaction est décrite dans la littérature pour des urées différemment substituées (Viret J. et al. Tetrahedron 1987, 43, 891-894 ; Murakami Y. et al. Chem. Pharm. Bull. 1983, 31, 423-428 ; Murakami Y. et Yokotama Y. Heterocycles 1979, 12, 1571-1574).

Ce réarrangement de Shestakov sera avantageusement réalisé en présence d'un agent inducteur de réarrangement choisi parmi NaOCl et KOCl, et de préférence étant NaOCl.

De préférence, l'agent inducteur de réarrangement est introduit de manière échelonnée dans le temps. Ainsi, l'agent inducteur pourra être introduit en plusieurs portions étalées dans le temps ou au goutte à goutte, ou encore en continu de manière lente pour que l'addition soit suffisamment étalée dans le temps. L'homme du métier saura adapter le paramètre d'addition (telle que la vitesse d'addition pour une addition en continu) de l'agent inducteur de manière à avoir une addition optimale.

Au moins un équivalent molaire, avantageusement entre 1 et 1,5 équivalents molaires, et de préférence environ 1 équivalent molaire, d'agent inducteur de réarrangement est utilisé dans cette réaction, pour un équivalent molaire d'urée de formule (III).

En effet, les inventeurs ont remarqué de manière surprenante que de meilleurs rendements étaient obtenus lorsque l'agent inducteur de réarrangement était ajouté progressivement au milieu réactionnel par rapport à un seul ajout.

Bien évidemment, le nombre de portions additionnées et la durée de l'addition dépendront de l'échelle à laquelle est effectuée la réaction, de telles adaptations étant de la pratique commune de l'homme du métier.

Le réarrangement de Shestakov pourra être réalisé dans un mélange dioxane/eau ou dans un alcool comme solvant, et de préférence dans le *tert*-butanol.

Par « alcool », on entend, au sens de la présente invention, un composé de formule Alk-OH, où Alk représente un groupe alkyle tel que défini ci-dessus. De préférence, il s'agit du méthanol, de l'éthanol ou du *tert*-butanol.

Ce réarrangement est réalisé avantageusement dans des conditions basiques, notamment en présence d'hydroxyde de sodium ou de potassium et de préférence, en présence d'hydroxyde de potassium.

Par ailleurs, l'urée de formule (III) telle que définie ci-dessus peut être préparée à partir de l'amine de formule (II) telle que définie ci-dessus selon les étapes suivantes :
(1) mise en réaction de l'amine de formule (II) avec l'isocyanate de trichloroacétyle (Cl₃CC(O)-N=C=O) pour donner le composé de formule (IV) : 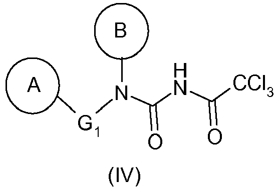 dans laquelle G₁, A et B sont tels que définis ci-dessus, et
(2) traitement en milieu basique du composé de formule (IV) obtenu à l'étape précédente pour donner une urée de formule (III).

L'étape (1) pourra être réalisée dans un solvant tel que l'acétonitrile, le tétrahydrofurane, le dichlorométhane ou encore le dioxane et de préférence dans l'acétonitrile.

L'étape (1) peut être réalisée à température ambiante, c'est-à-dire à des températures variant entre 20 et 40°C, et de préférence à environ 25°C.

L'hydroxyde de potassium ou de sodium, et de préférence KOH, pourra être utilisé comme base dans l'étape (2).

Le solvant utilisé dans cette étape (2) sera un alcool tel que défini ci-dessus et de préférence sera le méthanol, l'étape (2) étant réalisée notamment au reflux de ce solvant.

Ainsi, selon un mode de réalisation particulier du procédé de l'invention, l'hydrazine de formule (I) précédente pourra être préparée selon les étapes successives suivantes :
(1) mise en réaction d'une amine de formule (II) telle que définie précédemment avec Cl₃CC(O)-N=C=O pour donner un composé de formule (IV) tel que défini précédemment,
(2) traitement en milieu basique du composé de formule (IV) obtenu à l'étape précédente pour donner une urée de formule (III) telle que définie précédemment,
(3) réaction de Shestakov à partir de l'urée de formule (III) obtenue à l'étape précédente pour donner une hydrazine de formule (I) telle que définie précédemment, et
(4) isolation du milieu réactionnel de l'hydrazine de formule (I) obtenue à l'étape précédente.

L'isolation du produit final pourra être réalisée par des techniques bien connues de l'homme du métier, comme par exemple par extraction, évaporation du solvant ou encore par précipitation et filtration.

L'hydrazine ainsi obtenue pourra par ailleurs être purifiée si nécessaire par des méthodes bien connues de l'homme du métier, comme par recristallisation si le composé est cristallin, ou encore par distillation.

Avantageusement, G₁ représente une liaison ou une chaîne hydrocarbonée linéaire et saturée, comprenant 1 à 4 atomes de carbone, et de préférence représente une liaison.

De manière avantageuse, le radical A défini ci-dessus est un aryle, de préférence un phényle, substitué en méta ou en para, de préférence en para, par un groupement alkoxy, tel que méthoxy, ou par un groupement aryle ou arylalkyle, tel que phényle ou benzyle, éventuellement substitué par un ou deux substituants, identiques ou différents, tels qu'un groupement acyle ou alkoxy.

Avantageusement, B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle.

En particulier, l'hydrazine de formule (I) est la *N*-(4-benzyl-phényl)-*N*-(2-méthyl-6-pipéridin-1-yl-phényl)-hydrazine (hydrazine (Ia)).

Par conséquent, l'hydrazine (Ia) précédente est préparée à partir de 1-(4-benzyl-phényl)-1-(2-méthyl-6-pipéridin-1-yl-phényl)-urée (composé (IIIa)), elle-même préparée à partir de (4-benzyl-phényl)-(2-méthyl-6-pipéridin-1-yl-phényl)-amine (composé (IIa)).

La présente invention a également pour objet un procédé de préparation d'un composé de formule (V) suivante : 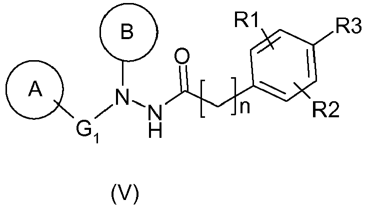 et de ses sels pharmaceutiquement acceptables,
dans lequel :
▪ R1 représente un groupement alkoxy, tel que méthoxy, de préférence en position ortho par rapport à R3,
▪ R2 représente un atome d'hydrogène ou d'halogène, tel que chlore ou brome, ou un groupement alkyle, tel que méthyle, de préférence en position méta par rapport à R3,
▪ R3 représente un groupement acide ou ester, et de préférence acide,
▪ n est un nombre entier compris entre 1 et 4, de préférence entre 1 et 2, et encore de préférence est 1,
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle, de préférence identiques,
▪A représente un groupement aryle, tel que phényle, éventuellement substitué :
   - en position méta ou para par :
      - un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
      - un groupement cycloalkyle, aryle ou arylalkyle, chacun éventuellement substitué par un ou deux substituants, identiques ou différents, tels qu'un groupement acyle ou alkoxy,
   - et/ou en position ortho ou méta par un groupement alkyle, et
▪ B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle,
caractérisé en ce qu'il comprend un procédé de préparation d'un composé de formule (I) tel que défini ci-dessus.

Les composés de formule (V) correspondent à des composés utiles pour le traitement du virus du papillome décrits dans la demande WO 2007/135 106. Cette demande de brevet décrit par ailleurs le procédé de préparation des composés de formule (V) à partir de l'hydrazine de formule (I), ainsi que le procédé de préparation des amines de formule (II).

Par « acide », on entend, au sens de la présente invention, un groupement COOH.

Par « ester », on entend, au sens de la présente invention, un groupement -CO-O-Alk, où Alk représente un groupement alkyle tel que défini précédemment.

Avantageusement, R2 représente un atome d'halogène, tel qu'un atome de brome, de préférence en position méta par rapport à R3.

Avantageusement, G₁ représente une liaison ou une chaîne hydrocarbonée linéaire et saturée, comprenant 1 à 4 atomes de carbone, et de préférence représente une liaison.

De manière avantageuse, le radical A défini ci-dessus est un aryle, de préférence un phényle, substitué en méta ou en para, de préférence en para, par un groupement alkoxy, tel que méthoxy, ou par un groupement aryle ou arylalkyle, tel que phényle ou benzyle, éventuellement substitué par un ou deux substituants, identiques ou différents, tels qu'un groupement acyle ou alkoxy.

Avantageusement, B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle.

De préférence, le composé de formule (V) répond aux caractéristiques suivantes :
- A représente un groupement phényle substitué en position para par un groupe benzyle,
- B représente un groupe phényle substitué en position ortho par un groupe pipéridin-1-yle et en position ortho' par un groupe méthyle,
- G₁ représente une liaison, et
- R1, R2, R3 et n sont tels que définis précédemment.

En particulier, le composé de formule (V) est le chlorhydrate de l'acide 4-[*N'-*(4-benzyl-phényl)-*N*'-(2-méthyl-6-pipéridin-1-yl-phényl)-hydrazinocarbonyl méthyl]-5-bromo-2-méthoxy-benzoïque (composé (Va)).

Par conséquent, le composé (Va) pourra être préparé à partir des intermédiaires (Ia), (IIa) et (IIIa) cités précédemment.

La présente invention a également pour objet un composé de formule générale (III) : 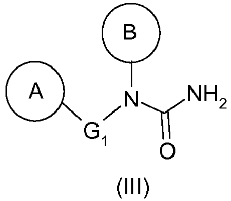 dans lequel :
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle, de préférence identiques,
▪ A représente un groupement aryle, tel que phényle, éventuellement substitué :
   - en position méta ou para par :
      - un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
      - un groupement cycloalkyle, aryle ou arylalkyle, chacun éventuellement substitué par un ou deux substituants, identiques ou différents, tels qu'un groupement acyle ou alkoxy,
   - et/ou en position ortho ou méta par un groupement alkyle, et
▪ B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle.

Avantageusement, G₁ représente une liaison ou une chaîne hydrocarbonée linéaire et saturée, comprenant 1 à 4 atomes de carbone, et de préférence représente une liaison.

De manière avantageuse, le radical A défini ci-dessus est un aryle, de préférence un phényle, substitué en méta ou en para, de préférence en para, par un groupement alkoxy, tel que méthoxy, ou par un groupement aryle ou arylalkyle, tel que phényle ou benzyle, éventuellement substitué par un ou deux substituants, identiques ou différents, tels qu'un groupement acyle ou alkoxy.

Avantageusement, B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle.

En particulier, le composé (III) sera la 1-(4-benzyl-phényl)-1-(2-méthyl-6-pipéridin-1-yl-phényl)-urée (composé (IIIa)).

La présente invention sera mieux comprise à la lumière des exemples non limitatifs qui suivent.

### EXEMPLE

### Abréviations utilisés dans les modes opératoires :

- CCM: Chromatographie sur Couche Mince
- éq.: Equivalent molaire
- HPLC: Chromatographie Liquide Haute Performance
- RMN: Résonance magnétique nucléaire
- SM: Spectrométrie de masse

### EXEMPLE : Synthèse du composé (Va)

### Stade 1 : Synthèse de l'amine (IIa)

Ce composé est synthétisé selon le mode opératoire décrit dans la demande WO 2007/135 106. Il correspond au composé obtenu au stade 3 de l'exemple 27 de cette demande (pages 90-91).

### Stade 2 : Synthèse de l'urée (IIIa)

### Etape (1)

A une suspension de l'amine (Ia) (4,00 g, 11,23 mmol, 1,0 éq.) dans l'acétonitrile anhydre (100mL), sous argon et sous agitation, est ajouté de l'isocyanate de trichloroacétyle (2,53 g, 13,48 mmol, 1,2 éq.) au goutte à goutte, à 18°C, pendant 10 min. La solution jaune limpide résultante est agitée pendant encore 15 min. à température ambiante. Après consommation complète de l'amine (IIa) (suivi par CCM et HPLC), le solvant est évaporé sous pression réduite. Le composé intermédiaire brut brun clair ainsi obtenu (6,2 g, rendement quantitatif) est utilisé directement dans l'étape suivante sans purification supplémentaire.
Rendement : quantitatif
HPLC : 92,24 %
RMN ¹H (CDCl₃, 200 MHz) δ (ppm) : 8,80 (s élargi, 1H), 6,95-7,31 (m, 12H), 3,92 (s, 2H), 2,86 (m, 2H), 2,73 (m, 2H), 2,03 (s, 3H), 1,53 (m, 6H)

### Etape (2)

A une solution de l'intermédiaire brut obtenu à l'étape (1) précédente (6,13 g, 11,2 mmol, 1,0 éq.) dans le méthanol (120 mL), est ajoutée une solution aqueuse de KOH (12,73g de KOH, 224,0 mmol, 20 éq., dans 25 mL d'eau) sous agitation. Le mélange réactionnel est alors chauffé à 75°C sous agitation pendant 1h30 à la même température. Après consommation complète de l'intermédiaire (suivi par CCM), le mélange réactionnel est concentré à sec. Le mélange brut résultant est alors repris dans de l'eau (100 mL) et extrait avec de l'acétate d'éthyle (3x100 mL). Les phases organiques rassemblées sont séchées sur sulfate de sodium, filtrées et concentrées sous pression réduite pour donner l'urée désirée (IIIa) (4,43 g, 11,1 mmol, 99 % de rendement) sous forme d'une gomme incolore avec une pureté de 83 % déterminée par HPLC. Cette urée est alors utilisée telle quelle dans l'étape suivante.
Rendement : 99 %
SM : MH⁺ 400
HPLC : 83,01 %
RMN ¹H (CDCl₃, 200 MHz) δ (ppm) : 6,94-7,30 (m, 12H), 4,77 (s, 2H), 3,91 (s, 2H), 2,91 (m, 2H), 2,60 (m, 2H), 2,26 (s, 3H), 1,48 (m, 6H).

### Stade 3 : Synthèse de l'hydrazine (Ia)

A une solution de l'urée brute (IIIa) obtenue au stade 2 précédent (4,43 g, 11,1 mmol, 1 éq.) dans le *tert*-butanol (50 mL) est ajoutée une solution aqueuse de KOH (12,58 g de KOH, 222,1 mmol, 20 éq., dans 25 mL d'eau) à 18°C, sous agitation, pendant 10 min. Au mélange réactionnel résultant, est ajoutée une solution aqueuse de NaOCl (8,21 mL, 11,1 mmol, 10 % de chlore actif) par portions (1 mL toutes les 15 min.) sur une période de 2h15. Après agitation vigoureuse à 18°C pendant 15 min. supplémentaires, les phases sont séparées. Le mélange réactionnel est extrait avec de l'acétate d'éthyle (3x100 mL). Les phases organiques rassemblées sont concentrées jusqu'à siccité sous pression réduite pour donner l'hydrazine désirée (4 g, 83 % de pureté déterminée par HPLC). Le résidu ainsi obtenu est alors purifié par chromatographie flash sur colonne sur gel de silice (éluant : cyclohexane/acétate d'éthyle 95/5) pour donner l'hydrazine désirée (3,4 g, 9,1 mmol, 81 % de rendement global) sous forme d'un solide de couleur crème avec une pureté de 95,6 % déterminée par HPLC.
Rendement global : 81 %
SM : MH⁺ 372
HPLC : 95,60 %
RMN ¹H (CDCl₃, 400 MHz) δ (ppm) : 7,25 (m, 2H), 7,14 (m, 4H), 6,97 (m, 4H), 6,73 (m, 2H), 4,83 (s élargi, 2H), 3,87 (s, 2H), 2,80 (s élargi, 4H), 2,08 (s, 3H), 1,46-1,57 (m, 6H).

### Stade 4 : Synthèse du composé (Va)

Ce composé est préparé à partir de l'hydrazine (Ia) obtenue au stade 3 précédent selon le protocole décrit dans la demande WO 2007/135 106. Ce protocole est décrit à l'exemple 27 de cette demande, et correspond aux stades 5 et 6 de cet exemple (pages 91-92).

## Revendications

1. Procédé de préparation d'un composé de formule générale (I) : 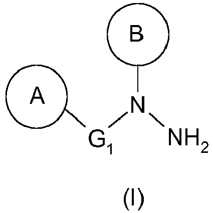 et de ses sels pharmaceutiquement acceptables,
dans lequel :
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle,
▪A représente un groupement aryle éventuellement substitué :
- en position méta ou para par :
• un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, ou
• un groupement cycloalkyle, aryle ou arylalkyle, chacun éventuellement substitué par un ou deux substituants, identiques ou différents,
- et/ou en position ortho ou méta par un groupement alkyle, et
▪ B représente un groupement aryle substitué en position ortho par un hétérocycle et éventuellement substitué en position ortho' par un groupement alkyle,
comprenant les étapes suivantes :
(1) mise en réaction d'une amine de formule (II) suivante : 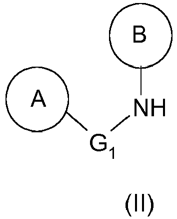 dans laquelle G₁, A et B sont tels que définis ci-dessus,
avec Cl₃CC(O)-N=C=O pour donner un composé de formule (IV) suivante : 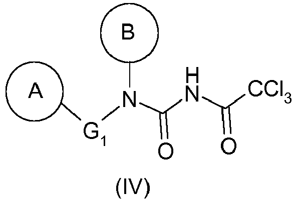 dans laquelle G₁, A et B sont tels que définis ci-dessus,
(2) traitement en milieu basique du composé de formule (IV) obtenu à l'étape précédente pour donner une urée de formule (III) suivante : 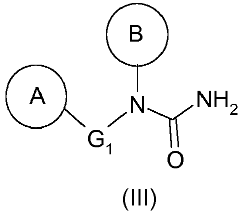 dans laquelle G₁, A et B sont tels que définis ci-dessus, et
(3) réarrangement de Shestakov de l'urée de formule (III) pour donner le composé de formule (I).

2. Procédé selon la revendication 1, **caractérisé en ce que** le réarrangement de Shestakov est réalisé en présence d'un agent inducteur de réarrangement choisi parmi NaOCl et KOC1, et de préférence étant NaOCl.

3. Procédé selon l'une quelconque des revendications 1 et 2, **caractérisé en ce que** l'agent inducteur de réarrangement est introduit de manière échelonnée dans le temps.

4. Procédé selon l'une quelconque des revendications 1 à 3, **caractérisé en ce que** :
▪ A représente un groupement phényle éventuellement substitué :
- en position méta ou para par :
• un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, choisis parmi un atome d'halogène, un groupement alkoxy et un groupement acyle, ou
• un groupement cycloalkyle, aryle ou arylalkyle, chacun éventuellement substitué par un ou deux substituants, identiques ou différents, choisis parmi un groupement acyle et un groupment alkoxy,
- et/ou en position ortho ou méta par un groupement alkyle, et
▪ B représente un groupement phényle substitué en position ortho par un N-cycloalkyle, tel qu'un groupement pipéridin-1-yle, et éventuellement substitué en position ortho' par un groupement alkyle.

5. Procédé selon l'une quelconque des revendications 1 à 4, **caractérisé en ce que** B représente un groupement phényle substitué en position ortho par un groupement pipéridin-1-yle et éventuellement substitué en position ortho' par un groupement méthyle.

6. Procédé selon l'une quelconque des revendications 1 à 5, **caractérisé en ce que** A représente un aryle, de préférence un phényle, substitué en méta ou en para, de préférence en para, par un groupement alkoxy tel que méthoxy ou par un groupement aryle ou arylalkyle éventuellement substitué par un ou deux substituants, identiques ou différents, tels qu'un groupement acyle ou alkoxy.

7. Procédé selon l'une quelconque des revendications 1 à 6, **caractérisé en ce que** le composé de formule (I) est la *N*-(4-benzyl-phényl)-*N*-(2-méthyl-6-pipéridin-1-yl-phényl)-hydrazine.

8. Procédé de préparation d'un composé de formule (V) suivante : 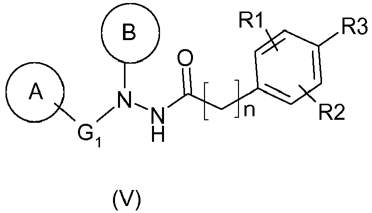 et de ses sels pharmaceutiquement acceptables,
dans lequel :
▪ R1 représente un groupement alkoxy, tel que méthoxy, de préférence en position ortho par rapport à R3,
▪ R2 représente un atome d'hydrogène ou d'halogène, tel que chlore ou brome, ou un groupement alkyle, tel que méthyle, de préférence en position méta par rapport à R3,
▪ R3 représente un groupement acide ou ester, et de préférence acide,
▪ n est un nombre entier compris entre 1 et 4, de préférence entre 1 et 2, et encore de préférence est 1,
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle, de préférence identiques,
▪ A représente un groupement aryle, tel que phényle, éventuellement substitué :
- en position méta ou para par :
• un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
• un groupement cycloalkyle, aryle ou arylalkyle, chacun éventuellement substitué par un ou deux substituants, identiques ou différents, tels qu'un groupement acyle ou alkoxy,
- et/ou en position ortho ou méta par un groupement alkyle, et
▪ B représente un groupement aryle, de préférence un phényle, substitué en position ortho par un hétérocycle, de préférence un N-cycloalkyle, tel qu'un groupement pipéridin-1-yle, et éventuellement substitué en position ortho' par un groupement alkyle, tel qu'un méthyle,
**caractérisé en ce que** le composé de formule (V) est préparé à partir d'un composé de formule (I) selon l'une quelconque des revendications 1 à 7 et le composé de formule (I) est préparé par un procédé selon l'une quelconque des revendications 1 à 7.

9. Procédé selon la revendication 8, **caractérisé en ce que** A représente un aryle, de préférence un phényle, substitué en méta ou en para, de préférence en para, par un groupement alkoxy tel que méthoxy ou par un groupement aryle ou arylalkyle éventuellement substitué par un ou deux substituants, identiques ou différents, tels qu'un groupement acyle ou alkoxy.

10. Procédé selon l'une quelconque des revendications 8 et 9, **caractérisé en ce que** le composé de formule (V) est le chlorhydrate de l'acide 4-[*N*'-(4-benzyl-phényl)-*N*'-(2-méthyl-6-pipéridin-1-yl-phényl)-hydrazinocarbonyl méthyl]-5-bromo-2-méthoxy-benzoïque.

11. Composé de formule générale (III) : 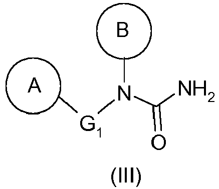 dans lequel :
▪ G₁ représente une liaison ou une chaîne hydrocarbonée, linéaire ou ramifiée, saturée ou insaturée, comprenant 1 à 4 atomes de carbone, éventuellement substituée par un ou deux groupements alkyle,
▪ A représente un groupement aryle éventuellement substitué :
- en position méta ou para par :
• un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, ou
• un groupement cycloalkyle, aryle ou arylalkyle, chacun éventuellement substitué par un ou deux substituants, identiques ou différents,
- et/ou en position ortho ou méta par un groupement alkyle, et
▪ B représente un groupement aryle substitué en position ortho par un hétérocycle et éventuellement substitué en position ortho' par un groupement alkyle.

12. Composé selon la revendication 11, **caractérisé en ce que** :
▪ A représente un groupement phényle éventuellement substitué :
- en position méta ou para par :
• un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, choisis parmi un atome d'halogène, un groupement alkoxy et un groupement acyle, ou
• un groupement cycloalkyle, aryle ou arylalkyle, chacun éventuellement substitué par un ou deux substituants, identiques ou différents, choisis parmi un groupement acyle et un groupment alkoxy,
- et/ou en position ortho ou méta par un groupement alkyle, et
▪ B représente un groupement phényle substitué en position ortho par un N-cycloalkyle, tel qu'un groupement pipéridin-1-yle, et éventuellement substitué en position ortho' par un groupement alkyle.

13. Composé selon l'une quelconque des revendications 11 et 12, **caractérisé en ce que** B représente un groupement phényle substitué en position ortho par un groupement pipéridin-1-yle et éventuellement substitué en position ortho' par un groupement méthyle.

14. Composé selon l'une quelconque des revendications 11 à 13, **caractérisé en ce que** A représente un aryle, de préférence un phényle, substitué en méta ou en para, de préférence en para, par un groupement alkoxy tel que méthoxy ou par un groupement aryle ou arylalkyle éventuellement substitué par un ou deux substituants, identiques ou différents, tels qu'un groupement acyle ou alkoxy.

15. Composé selon l'une quelconque des revendications 11 à 14, **caractérisé en ce qu'**il s'agit de la 1-(4-benzyl-phényl)-1-(2-méthyl-6-pipéridin-1-yl-phényl)-urée.

## Patentansprüche

1. Verfahren zur Herstellung einer Verbindung der allgemeinen Formel (I) : 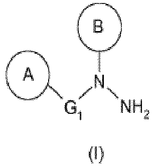 und von ihren pharmazeutisch verträglichen Salzen, in der
▪ G₁ für eine Bindung oder eine geradkettige oder verzweigte, gesättigte oder ungesättigte Kohlenwasserstoffkette mit 1 bis 4 Kohlenstoffatomen steht, die gegebenenfalls durch eine oder zwei Alkylgruppen substituiert ist,
▪ A für eine Arylgruppe steht, die gegebenenfalls substituiert ist:
- in meta- oder para-Stellung durch:
• ein Halogenatom oder eine Cyano-, Alkoxy-, Halogenalkoxy-, Acylaminoalkyl- oder -XR-Gruppe, wobei X für -O-, -S-, -SO-, -SO₂- oder -CO- steht und R für eine Arylalkyl-, Cycloalkyl- oder Arylgruppe steht, jede gegebenenfalls durch einen oder zwei gleiche oder verschiedene Substituenten substituiert, oder
• eine Cycloalkyl-, Aryl- oder Arylalkylgruppe, jede gegebenenfalls durch einen oder zwei gleiche oder verschiedene Substituenten substituiert,
- und/oder in ortho- oder meta-Stellung durch eine Alkylgruppe, und
▪ B für eine Arylgruppe steht, die in ortho-Stellung durch einen Heterozyklus substituiert ist, und gegebenenfalls in ortho'-Stellung durch eine Alkylgruppe substituiert ist,
umfassend die folgenden Schritte:
(1) Umsetzen eines Amins der folgenden Formel (II) : 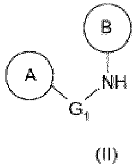 in der G₁, A und B wie oben definiert sind,
mit Cl₃CC (O) -N=C=O, um eine Verbindung der folgenden Formel (IV) zu ergeben: 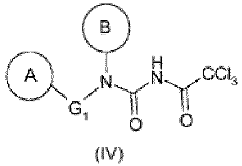 in der G₁, A und B wie oben definiert sind,
(2) Behandeln der im vorhergehenden Schritt erhaltenen Verbindung der Formel (IV) in basischem Medium, um einen Harnstoff der folgenden Formel (III) zu ergeben: 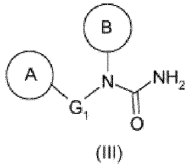 in der G₁, A und B wie oben definiert sind, und
(3) Shestakov-Umlagerung des Harnstoffs der Formel (III), um die Verbindung der Formel (I) zu ergeben.

2. Verfahren nach Anspruch 1, **dadurch gekennzeichnet, dass** die Shestakov-Umlagerung in Gegenwart eines die Umlagerung induzierenden Mittels erfolgt, ausgewählt aus NaOCl und KOCl, und wobei es sich bevorzugt um NaOCl handelt.

3. Verfahren nach einem der Ansprüche 1 und 2, **dadurch gekennzeichnet, dass** das die Umlagerung induzierende Mittel in zeitlich gestaffelter Weise eingeführt wird.

4. Verfahren nach einem der Ansprüche 1 bis 3, **dadurch gekennzeichnet, dass**:
▪ A für eine Phenylgruppe steht, die gegebenenfalls substituiert ist:
- in meta- oder para-Stellung durch:
• ein Halogenatom oder eine Cyano-, Alkoxy-, Halogenalkoxy-, Acylaminoalkyl- oder -XR-Gruppe, wobei X für -O-, -S-, -SO-, -SO₂- oder -CO- steht und R für eine Arylalkyl-, Cycloalkyl- oder Arylgruppe steht, jede gegebenenfalls durch einen oder zwei gleiche oder verschiedene Substituenten substituiert, ausgewählt aus einem Halogenatom, einer Alkoxygruppe und einer Acylgruppe, oder
• eine Cycloalkyl-, Aryl- oder Arylalkylgruppe, jede gegebenenfalls durch einen oder zwei gleiche oder verschiedene Substituenten substituiert, ausgewählt aus einer Acylgruppe und einer Alkoxygruppe,
- und/oder in ortho- oder meta-Stellung durch eine Alkylgruppe, und
▪ B für eine Phenylgruppe steht, die in ortho-Stellung durch ein N-Cycloalkyl, wie beispielsweise eine Piperidin-1-yl-Gruppe substituiert ist, und gegebenenfalls in ortho'-Stellung durch eine Alkylgruppe substituiert ist.

5. Verfahren nach einem der Ansprüche 1 bis 4, **dadurch gekennzeichnet, dass** B für eine Phenylgruppe steht, die in ortho-Stellung durch eine Piperidin-1-yl-Gruppe substituiert ist, und gegebenenfalls in ortho'-Stellung durch eine Methylgruppe substituiert ist.

6. Verfahren nach einem der Ansprüche 1 bis 5, **dadurch gekennzeichnet, dass** A für ein Aryl, bevorzugt ein Phenyl steht, das in meta oder in para, bevorzugt in para, durch eine Alkoxy-, wie beispielsweise Methoxygruppe oder durch eine Aryl- oder Arylalkylgruppe substituiert ist, die gegebenenfalls durch einen oder zwei gleiche oder verschiedene Substituenten, wie beispielsweise eine Acyl- oder Alkoxygruppe substituiert ist.

7. Verfahren nach einem der Ansprüche 1 bis 6, **dadurch gekennzeichnet, dass** es sich bei der Verbindung der Formel (I) um *N*-(4-Benzyl-phenyl)-*N*-(2-methyl-6-piperidin-1-yl-phenyl)-hydrazin handelt.

8. Verfahren zur Herstellung einer Verbindung der folgenden Formel (V): 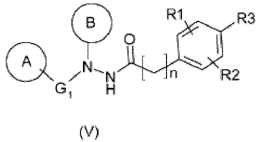 und von ihren pharmazeutisch verträglichen Salzen, in der
▪ R1 für eine Alkoxy-, wie beispielsweise Methoxygruppe, bevorzugt in ortho-Stellung bezogen auf R3 steht,
▪ R2 für ein Wasserstoff- oder Halogenatom, wie beispielsweise Chlor oder Brom, oder eine Alkyl-, wie beispielsweise Methylgruppe, bevorzugt in meta-Stellung bezogen auf R3 steht,
▪ R3 für eine Säure- oder Ester-, und bevorzugt Säuregruppe steht,
▪ n eine Ganzzahl im Bereich zwischen 1 und 4, bevorzugt zwischen 1 und 2 ist, und noch bevorzugter 1 ist,
▪ G₁ für eine Bindung oder eine geradkettige oder verzweigte, gesättigte oder ungesättigte Kohlenwasserstoffkette mit 1 bis 4 Kohlenstoffatomen steht, die gegebenenfalls durch eine oder zwei, bevorzugt gleiche, Alkylgruppen substituiert ist,
▪ A für eine Aryl-, wie beispielsweise Phenylgruppe steht, die gegebenenfalls substituiert ist:
- in meta- oder para-Stellung durch:
• ein Halogenatom oder eine Cyano-, Alkoxy-, Halogenalkoxy-, Acylaminoalkyl- oder -XR-Gruppe, wobei X für -O-, -S-, -SO-, -SO₂- oder -CO- steht und R für eine Arylalkyl-, Cycloalkyl- oder Arylgruppe steht, jede gegebenenfalls durch einen oder zwei gleiche oder verschiedene Substituenten, wie beispielsweise Halogenatom, Alkoxy- oder Acylgruppe substituiert, oder
• eine Cycloalkyl-, Aryl- oder Arylalkylgruppe, jede gegebenenfalls durch einen oder zwei gleiche oder verschiedene Substituenten, wie beispielsweise eine Acyl- oder Alkoxygruppe substituiert,
- und/oder in ortho- oder meta-Stellung durch eine Alkylgruppe, und
▪ B für eine Aryl-, bevorzugt eine Phenylgruppe steht, die in ortho-Stellung durch einen Heterozyklus, bevorzugt ein N-Cycloalkyl, wie beispielsweise eine Piperidin-1-yl-gruppe substituiert ist, und gegebenenfalls in ortho'-Stellung durch eine Alkyl-, wie beispielsweise Methylgruppe substituiert ist,
**dadurch gekennzeichnet, dass** die Verbindung der Formel (V) aus einer Verbindung der Formel (I) nach einem der Ansprüche 1 bis 7 hergestellt wird und die Verbindung der Formel (I) durch ein Verfahren nach einem der Ansprüche 1 bis 7 hergestellt wird.

9. Verfahren nach Anspruch 8, **dadurch gekennzeichnet, dass** A für ein Aryl, bevorzugt ein Phenyl steht, das in meta oder in para, bevorzugt in para, durch eine Alkoxy-, wie beispielsweise Methoxygruppe oder durch eine Aryl- oder Arylalkylgruppe substituiert ist, die gegebenenfalls durch einen oder zwei gleiche oder verschiedene Substituenten, wie beispielsweise eine Acyl- oder Alkoxygruppe substituiert ist.

10. Verfahren nach einem der Ansprüche 8 und 9, **dadurch gekennzeichnet, dass** es sich bei der Verbindung der Formel (V) um das Hydrochlorid der 4-[*N'*-(4-Benzyl-phenyl)-*N'*-(2-methyl-6-piperidin-1-yl-phenyl)hydrazincarbonylmethyl]-5-brom-2-methoxybenzoesäure handelt.

11. Verbindung der allgemeinen Formel (III) : 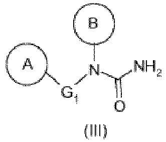 in der:
▪ G₁ für eine Bindung oder eine geradkettige oder verzweigte, gesättigte oder ungesättigte Kohlenwasserstoffkette mit 1 bis 4 Kohlenstoffatomen steht, die gegebenenfalls durch eine oder zwei Alkylgruppen substituiert ist,
▪ A für eine Arylgruppe steht, die gegebenenfalls substituiert ist:
- in meta- oder para-Stellung durch:
• ein Halogenatom oder eine Cyano-, Alkoxy-, Halogenalkoxy-, Acylaminoalkyl- oder -XR-Gruppe, wobei X für -O-, -S-, -SO-, -SO₂- oder -CO- steht und R für eine Arylalkyl-, Cycloalkyl- oder Arylgruppe steht, jede gegebenenfalls durch einen oder zwei gleiche oder verschiedene Substituenten substituiert, oder
• eine Cycloalkyl-, Aryl- oder Arylalkylgruppe, jede gegebenenfalls durch einen oder zwei gleiche oder verschiedene Substituenten substituiert,
- und/oder in ortho- oder meta-Stellung durch eine Alkylgruppe, und
▪ B für eine Arylgruppe steht, die in ortho-Stellung durch einen Heterozyklus substituiert ist, und gegebenenfalls in ortho'-Stellung durch eine Alkylgruppe substituiert ist.

12. Verbindung nach Anspruch 11, **dadurch gekennzeichnet, dass**
▪ A für eine Phenylgruppe steht, die gegebenenfalls substituiert ist:
- in meta- oder para-Stellung durch:
• ein Halogenatom oder eine Cyano-, Alkoxy-, Halogenalkoxy-, Acylaminoalkyl- oder -XR-Gruppe, wobei X für -O-, -S-, -SO-, -SO₂- oder -CO- steht und R für eine Arylalkyl-, Cycloalkyl- oder Arylgruppe steht, jede gegebenenfalls durch einen oder zwei gleiche oder verschiedene Substituenten substituiert, ausgewählt aus einem Halogenatom, einer Alkoxygruppe und einer Acylgruppe, oder
• eine Cycloalkyl-, Aryl- oder Arylalkylgruppe, jede gegebenenfalls durch einen oder zwei gleiche oder verschiedene Substituenten substituiert, ausgewählt aus einer Acylgruppe und einer Alkoxygruppe,
- und/oder in ortho- oder meta-Stellung durch eine Alkylgruppe, und
▪ B für eine Phenylgruppe steht, die in ortho-Stellung durch ein N-Cycloalkyl, wie beispielsweise eine Piperidin-1-yl-Gruppe substituiert ist, und gegebenenfalls in ortho'-Stellung durch eine Alkylgruppe substituiert ist.

13. Verbindung nach einem der Ansprüche 11 und 12, **dadurch gekennzeichnet, dass** B für eine Phenylgruppe steht, die in ortho-Stellung durch eine Piperidin-1-yl-Gruppe substituiert ist, und gegebenenfalls in ortho'-Stellung durch eine Methylgruppe substituiert ist.

14. Verbindung nach einem der Ansprüche 11 bis 13, **dadurch gekennzeichnet, dass** A für ein Aryl, bevorzugt ein Phenyl steht, das in meta oder in para, bevorzugt in para, durch eine Alkoxy-, wie beispielsweise Methoxygruppe oder durch eine Aryl- oder Arylalkylgruppe substituiert ist, die gegebenenfalls durch einen oder zwei gleiche oder verschiedene Substituenten, wie beispielsweise eine Acyl- oder Alkoxygruppe substituiert ist.

15. Verbindung nach einem der Ansprüche 11 bis 14, **dadurch gekennzeichnet, dass** es sich um 1-(4-Benzyl-phenyl)-1-(2-methyl-6-piperidin-1-yl-phenyl)-Harnstoff handelt.

## Claims

1. A method for producing a compound of general formula (I): 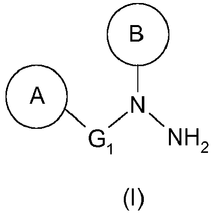 and the pharmaceutically acceptable salts thereof,
wherein:
▪ G₁ is a bond or a linear or branched, saturated or unsaturated hydrocarbon chain comprising 1 to 4 carbon atoms, optionally substituted by one or two alkyl groups,
▪ A is an aryl group, optionally substituted:
- in the meta or para position by:
• a halogen atom or a cyano, alkoxy, haloalkoxy, acylaminoalkyl or -XR group where X is -O-, -S-, -SO-, -SO₂- or -CO- and R is an arylalkyl, cycloalkyl or aryl group, each optionally substituted by one or two identical or different substituents, or
• a cycloalkyl, aryl or arylalkyl group, each optionally substituted by one or two identical or different substituents,
- and/or in the ortho or meta position by an alkyl group, and
▪ B is an aryl group, substituted in the ortho position by a heterocycle, and optionally substituted in the ortho' position by an alkyl group,
comprising the following steps :
(1) reaction of an amine of following formula (II): 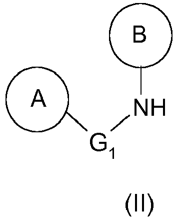 wherein G1, A and B are as defined above,
with Cl₃CC(O)-N=C=O to give a compound of following formula (IV): 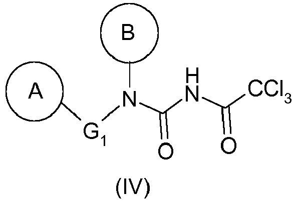 wherein G1, A and B are as defined above,
(2) treatment in basic medium of the compound of formula (IV) obtained in the preceding step to give a urea of following formula (III): 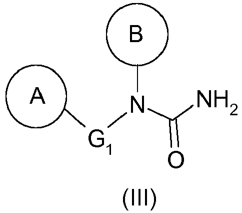 wherein G1, A and B are as defined above, and
(3) Shestakov rearrangement of the urea of formula (III) to give the compound of formula (I).

2. The method according to claim 1, **characterized in that** the Shestakov rearrangement is carried out in the presence of a rearrangement inducing agent selected from NaOCl and KOCl, and preferably being NaOCl.

3. The method according to any one of claims 1 and 2, **characterized in that** the rearrangement inducing agent is introduced in a staggered fashion.

4. The method according to any one of claims 1 to 3, **characterized in that**:
▪ A is a phenyl group optionally substituted:
- in the meta or para position by:
• a halogen atom or a cyano, alkoxy, haloalkoxy, acylaminoalkyl or -XR group where X is -O-, -S-, -SO-, -SO₂- or -CO- and R is an arylalkyl, cycloalkyl or aryl group, each optionally substituted by one or two identical or different substituents, selected from a halogen atom, an alkoxy group and an acyl group, or
• a cycloalkyl, aryl or arylalkyl group, each optionally substituted by one or two identical or different substituents, selected from an acyl group and an alkoxy group,
- and/or in the ortho or meta position by an alkyl group, and
▪ B is a phenyl group substituted in the ortho position by a N-cycloalkyl, such as a piperidin-1-yl group, and optionally substituted in the ortho' position by an alkyl group.

5. The method according to any one of claims 1 to 4, **characterized in that** B is a phenyl group substituted in the ortho position by a piperidin-1-yl group and optionally substituted in the ortho' position by a methyl group.

6. The method according to any one of claims 1 to 5, **characterized in that** A is an aryl, preferably a phenyl, substituted in the meta or para position, preferably in the para position, by an alkoxy group such as methoxy or by an aryl or arylalkyl group optionally substituted by one or two identical or different substituents, such as an acyl or alkoxy group.

7. The method according to any one of claims 1 to 6, **characterized in that** the compound of formula (I) is *N*-(4-benzyl-phenyl)-*N*-(2-methyl-6-piperidin-1-yl-phenyl)-hydrazine.

8. A method for producing a compound of following formula (V): 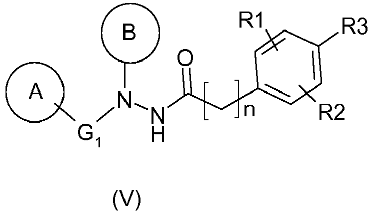 and the pharmaceutically acceptable salts thereof,
wherein:
▪ R1 is an alkoxy group, such as methoxy, preferably in the ortho position relative to R3,
▪ R2 is a hydrogen or halogen atom, such as chlorine or bromine, or an alkyl group, such as methyl, preferably in the meta position relative to R3,
▪ R3 is an acid or ester group, and preferably an acid group,
▪ n is an integer between 1 and 4, preferably between 1 and 2, and more preferably is 1,
▪ G₁ is a bond or a linear or branched, saturated or unsaturated hydrocarbon chain comprising 1 to 4 carbon atoms, optionally substituted by one or two preferably identical alkyl groups,
▪ A is an aryl group, such as phenyl, optionally substituted:
- in the meta or para position by:
• a halogen atom or a cyano, alkoxy, haloalkoxy, acylaminoalkyl or -XR group where X is -O-, -S-, -SO-, -SO₂- or -CO- and R is an arylalkyl, cycloalkyl or aryl group, each optionally substituted by one or two identical or different substituents, such as a halogen atom, an alkoxy or acyl group, or
• a cycloalkyl, aryl or arylalkyl group, each optionally substituted by one or two identical or different substituents, such as an acyl or alkoxy group,
- and/or in the ortho or meta position by an alkyl group, and
▪ B is an aryl group, preferably a phenyl, substituted in the ortho position by a heterocycle, preferably an N-cycloalkyl, such as a piperidin-1-yl group, and optionally substituted in the ortho' position by an alkyl group, such as a methyl,
**characterized in that** the compound of formula (V) is prepared from a compound of formula (I) according to any one of claims 1 to 7 and the compound of formula (I) is prepared by a method according to any one of claims 1 to 7.

9. The method according to claim 8, **characterized in that** A is an aryl, preferably a phenyl, substituted in the meta or para position, preferably in the para position, by an alkoxy group such as methoxy or by an aryl or arylalkyl group optionally substituted by one or two identical or different substituents, such as an acyl or alkoxy group.

10. The method according to any one of claims 8 and 9, **characterized in that** the compound of formula (V) is 4-[*N'*-(4-benzyl-phenyl)-*N'*-(2-methyl-6-piperidin-1-yl-phenyl)-hydrazinocarbonyl methyl]-5-bromo-2-methoxy benzoic acid hydrochloride.

11. A compound of general formula (III): 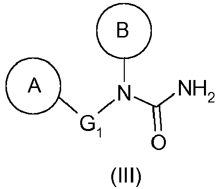 wherein:
▪ G₁ is a bond or a linear or branched, saturated or unsaturated hydrocarbon chain comprising 1 to 4 carbon atoms, optionally substituted by one or two alkyl groups,
▪ A is an aryl group, optionally substituted:
- in the meta or para position by:
• a halogen atom or a cyano, alkoxy, haloalkoxy, acylaminoalkyl or -XR group where X is -O-, -S-, -SO-, -SO₂- or -CO- and R is an arylalkyl, cycloalkyl or aryl group, each optionally substituted by one or two identical or different substituents, or
• a cycloalkyl, aryl or arylalkyl group, each optionally substituted by one or two identical or different substituents,
- and/or in the ortho or meta position by an alkyl group, and
▪ B is an aryl group substituted in the ortho position by a heterocycle, and optionally substituted in the ortho' position by an alkyl group.

12. The compound according to claim 11, **characterized in that**:
▪ A is a phenyl group optionally substituted:
- in the meta or para position by:
• a halogen atom or a cyano, alkoxy, haloalkoxy, acylaminoalkyl or -XR group where X is -O-, -S-, -SO-, -SO₂- or -CO- and R is an arylalkyl, cycloalkyl or aryl group, each optionally substituted by one or two identical or different substituents, selected from a halogen atom, an alkoxy group and an acyl group, or
• a cycloalkyl, aryl or arylalkyl group, each optionally substituted by one or two identical or different substituents, selected from an acyl group and an alkoxy group,
- and/or in the ortho or meta position by an alkyl group, and
▪ B is a phenyl group substituted in the ortho position by a N-cycloalkyl, such as a piperidin-1-yl group, and optionally substituted in the ortho' position by an alkyl group,

13. The compound according to any one of claims 11 and 12, **characterized in that** B is a phenyl group substituted in the ortho position by a piperidin-1-yl group and optionally substituted in the ortho' position by a methyl group.

14. The compound according to any one of claims 11 to 13, **characterized in that** A is an aryl, preferably a phenyl, substituted in the meta or para position, preferably in the para position, by an alkoxy group such as methoxy or by an aryl or arylalkyl group optionally substituted by one or two identical or different substituents, such as an acyl or alkoxy group.

15. The compound according to any one of claims 11 to 14, **characterized in that** it is 1-(4-benzyl-phenyl)-1-(2-methyl-6-piperidin-1-yl-phenyl)-urea.
